# EUROPÄISCHE PATENTSCHRIFT

(11) **EP 0 493 629 B1**
(45) Veröffentlichungstag und Bekanntmachung des Hinweises auf die Patenterteilung: **15.05.1996**
(21) Anmeldenummer: 90125819.4
(22) Anmeldetag: 31.12.1990
(51) Int. Cl.: A61F 2/38

(54) **Ellbogengelenk-Endoprothese**
Elbow endoprosthesis
Endoprothèse de coude

(43) Veröffentlichungstag der Anmeldung: 08.07.1992
(73) Patentinhaber: GMT Gesellschaft für medizinische Technik mbH, D-22767 Hamburg (DE); Waldemar Link (GmbH & Co.), D-22315 Hamburg (DE)
(72) Erfinder: Engelbrecht, Eckart, Dr.med., W-2000 Hamburg 60 (DE); Keller, Arnold, W-2061 Kayhude (DE)
(74) Vertreter: Heldt, Gert, Dr. Dipl.-Ing.

(56) Entgegenhaltungen:
- EP-A- 0 098 466
- EP-A- 0 321 927
- EP-A- 0 359 457
- AT-B- 331 384
- DE-A- 2 063 547
- FR-A- 2 129 100
- US-A- 3 990 117
- US-A- 4 383 337

## Beschreibung

Die Erfindung betrifft eine Ellenbogengelenk-Endoprothese mit einem Humerusteil, um das in einem Schwenklager (4) verschwenkbar ein Ulnateil (2) gelagert ist, an dem eine als Pfanne (6) ausgebildete Lagerung (5) befestigt ist, in der ein Radiusteil (3) mit einem Radiuskopf (7) gelagert ist.

Bei dieser aus der französischen Patentschrift 2129100 bekannt gewordenen Ellenbogengelenk-Endoprothese greift ein etwa kugelförmiger Kopf eines Radiusteils in eine Hülse ein, in der er relativ eng geführt ist. Zur Begrenzung von Längsbewegungen des Kopfes innerhalb der Hülse erstreckt sich durch den Innenraum der Hülse ein Sicherungsring, der in einer durch die Wandungen verlaufenden Nut geführt wird.

Ein den Kopf tragender Ulnateil kann innerhalb der Hülse nur begrenzt Schwenkbewegungen ausführen. Die Begrenzung dieser Bewegungen erfolgt dadurch, daß der Ulnateil mit seinem Schaft gegen die Wandungen der Hülse anschlägt, wenn der Schaft Schwenkbewegungen ausführt.

Darüber hinaus ist die Führung des Kopfteils an dem Sicherungsring mangelhaft. Einerseits wird der Kopf auf den scharfen Kanten des Sicherungsringes geführt, so daß die Bewegungen entsprechend wenig gleitend aber dafür relativ ruckhaft erfolgen.

Darüber hinaus muß damit gerechnet werden, daß die scharfen Kanten des Sicherungsringes von der Oberfläche des kugelförmigen Kopfes dünne Metallspäne abreiben, die vom menschlichen Körper aufgenommen werden müssen.

Aufgabe der vorliegenden Erfindung ist es daher, eine Ellenbogengelenk-Endoprothese der einleitend genannten Art so zu verbessern, daß sie während eines langen Nutzungszeitraumes ihre Funktionsfähigkeit ohne wesentliche Belastungen für den Patienten beibehält.

Diese Aufgabe wird erfindungsgemäß dadurch gelöst, daß die Pfanne (6) eine derart größere flächenhafte Ausdehnung als der von ihr umschlossene Bereich des Radiuskopfes (7) aufweist, daß der Radiuskopf (7) im Bereich der Pfanne (6) sowohl in Richtung auf einen im Ulnateil (2) verlaufenden Ulnaschaft (10) als auch quer zu dieser Richtung beweglich ist.

Diese Lagerung im Bereich des Ulnateils ermöglicht eine Realisierung von Bewegungsabläufen, die den natürlichen Verhältnissen weitgehend entsprechen. Während der Schwenkbewegungen des Ulnateils werden Relativbewegungen des Radiusteils gegenüber dem Ulnateil aufgrund der Ausbildung der Pfanne ermöglicht, wenn ein mit dem Ellenbogengelenk versehener Unterarm Schwenkbewegungen um seine Längsachse ausführt.

Dabei erfolgt eine sanfte Führung des Kugelkopfes auf der Pfannenoberfläche. Eine Begrenzung der Bewegungen findet aufgrund der Pfannenausbildung nicht statt.

Gemäß einer bevorzugten Ausführungsform weist ein Schaft des Humerusteils eine seitliche Neigung gegenüber einer von einem Schwenklager vorgegebenen Schwenkebene auf. Darüber hinaus ist der Schaft des Radiusteils mit einem Abstand zum Ulnateil und gleichfalls versetzt zur Längsachse des Humerusteils angeordnet. Dieser Versatz des Schäfte ermöglicht eine Ausrichtung von die Schäfte aufnehmenden Knochen in weitgehender Annäherung an die natürlichen anatomischen Verhältnisse. Diese den natürlichen anatomischen Verhältnissen angepaßte Anordnung von Humerus, Ulna und Radius vermeidet eine Schädigung des Bänderapparates durch Überlastungen. Darüber hinaus entspricht das äußere Erscheinungsbild eines menschlichen Arms nach Implantation der erfindungsgemäßen Endoprothese weitgehend dem natürlichen Aussehen.

Gemäß einer anderen bevorzugten Ausführungsform weist das Humerusteil im Bereich des Schwenklagers eine das Ulnateil führende Welle auf, die vom Ulnateil im wesentlichen U-förmig beaufschlagt ist. Diese Ausbildung des Schwenklagers ermöglicht die zunächst separate Implantation von Ulna-, Radius- und Humerusteil in die diese jeweils aufnehmenden Knochen und das nachträgliche Zusammenfügen der Prothesenteile. Der operierende Chirurg wird hierdurch weitgehend von Behinderungen durch einzelne Prothesenteile entlastet.

Weitere Einzelheiten der vorliegenden Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

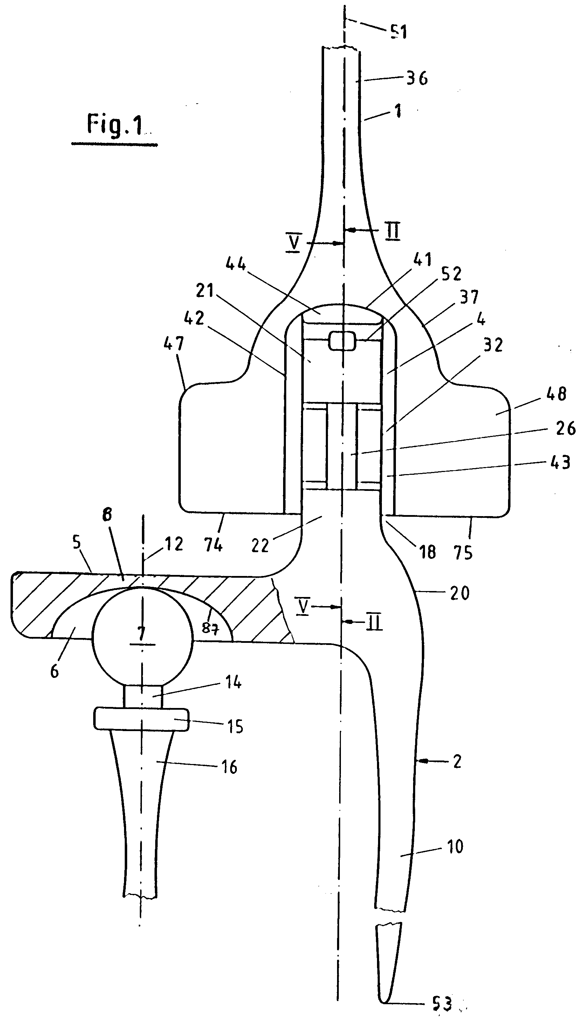

- Fig. 1:: eine teilweise geschnittene Vorderansicht einer Ellenbogengelenk-Endoprothese,

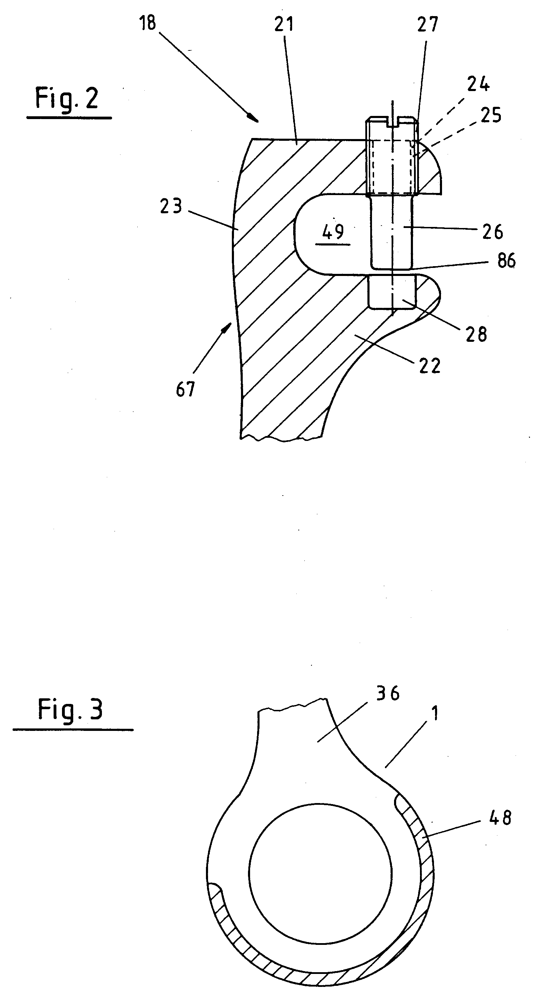

- Fig. 2:: eine teilweise Darstellung eines Schnittes durch das Ulnateil gemäß Schnittlinie II-II in Figur 1,
- Fig. 3:: eine teilweise Darstellung einer Seitenansicht des Humerusteils,

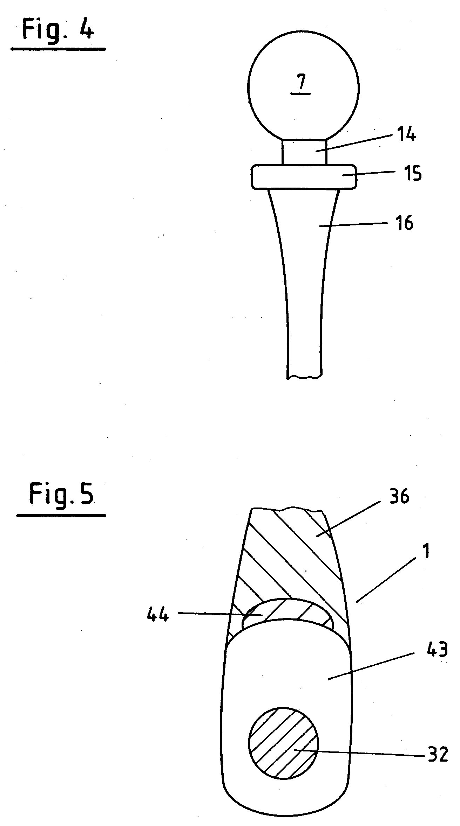

- Fig. 4:: eine teilweise Darstellung des Radiusteils,
- Fig. 5:: eine teilweise Darstellung eines Längsschnittes durch das Humerusteil gemäß Schnittlinie V-V in Figur 1,

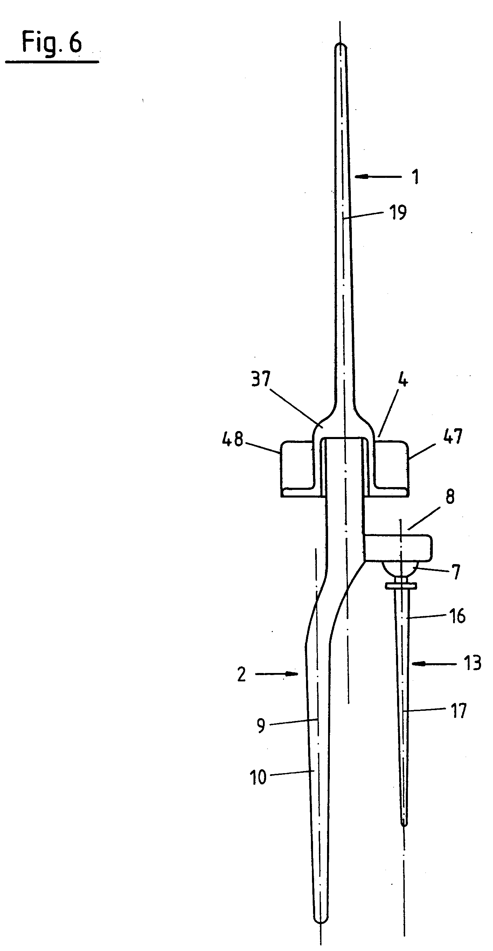

- Fig. 6:: eine Ansicht der Prothese gemäß Figur 1 von hinten,

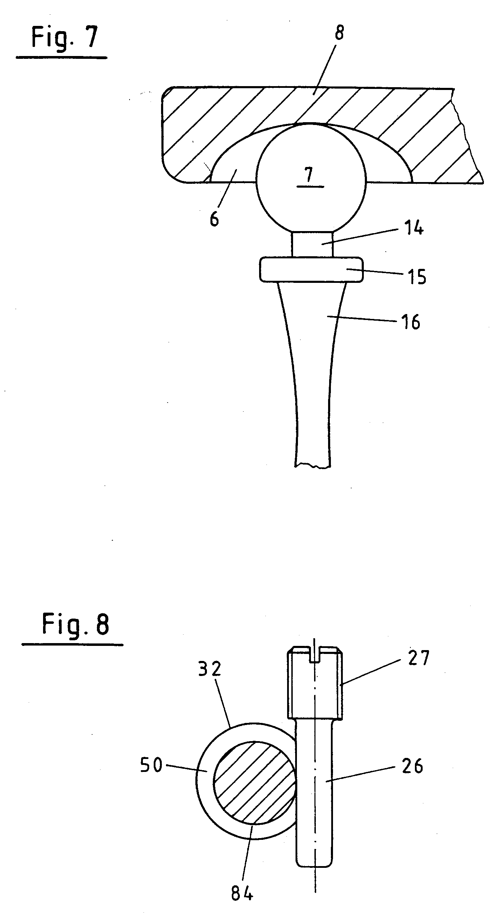

- Fig. 7:: eine teilweise Darstellung eines Schnittes durch eine den Radiuskopf spielbehaftet führende Pfanne,
- Fig. 8:: einen Längsschnitt durch eine von einer Sicherungsschraube beaufschlagte Welle mit runder Ausnehmung,

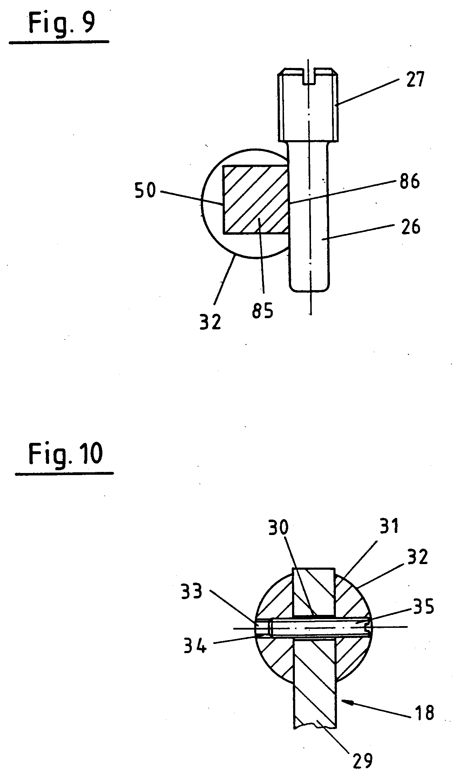

- Fig. 9:: einen Querschnitt durch eine von einer Sicherungsschraube beaufschlagte Welle mit kantiger Ausnehmung,
- Fig. 10:: einen Längsschnitt durch eine Welle, die eine Ausnehmung aufweist, die einen mit einer Madenschraube gesicherten Zapfen des Ulnateils aufnimmt,

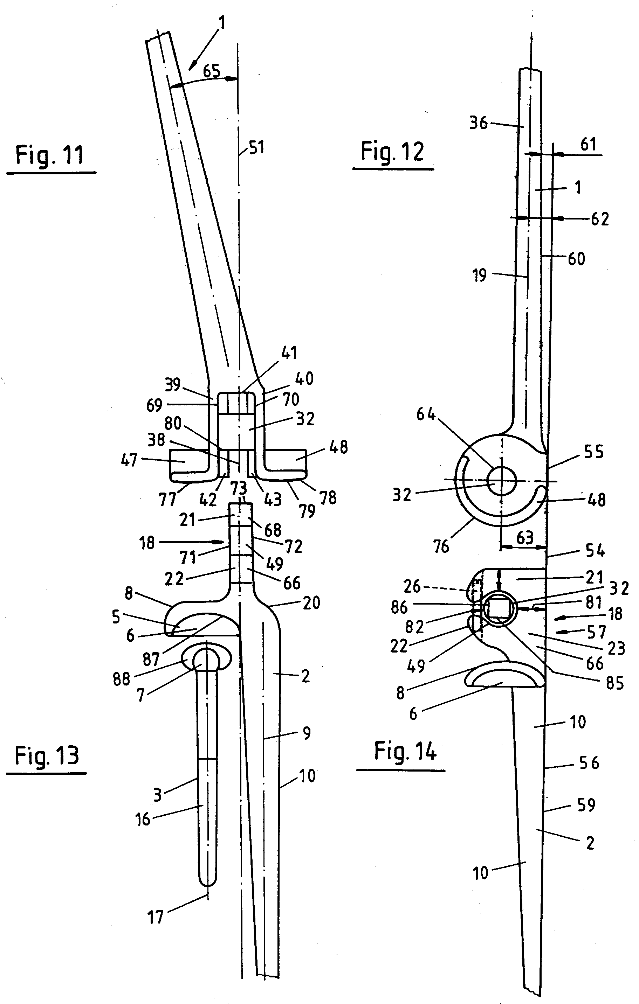

- Fig. 11:: eine Vorderansicht eines anderen Humerusteiles,
- Fig. 12:: eine Seitenansicht eines Humerusteiles nach Figur 11,
- Fig. 13:: eine Vorderansicht eines Ulnateiles mit aus der Pfanne herausgenommenem Radiusteil,
- Fig. 14:: eine Seitenansicht des eine Welle beaufschlagenden Ulnateils nach Figur 13,

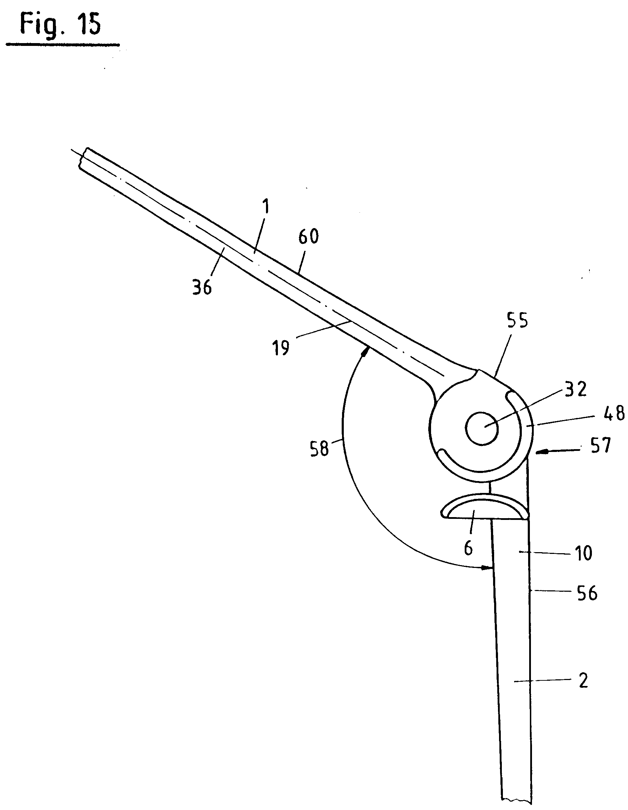

- Fig. 15:: eine Seitenansicht einer verschwenkten Ellenbogenge lenk-Endoprothese.

Eine Ellenbogengelenk-Endoprothese besteht im wesentlichen aus einem Humerusteil (1), einem im Bereich des Humerusteils (1) geführten Ulnateil (2) sowie einem im Bereich des Ulnateils (2) gelagerten Radiusteil (3). Die Führung des Ulnateiles (2) im Bereich des Humerusteils (1) ist als Schwenklager (4) ausgebildet. Dieses Schwenklager (4) ist an einem dem Humerusteil (1) zugewandten oberen Ende (52) des Ulnateils (2) befestigt. Um das Schwenklager (4) ist der Ulnateil (2) bezüglich des Humerusteils (1) in einer Schwenkebene (51) verschwenkbar gelagert. Vom Schwenklager (4) aus erstreckt sich ein Ulnaschaft (10) in eine vom Humerusteil (1) abgewandte Richtung, während sich ein Humerusschaft (36) in eine vom Ulnateil (2) abgewandte Richtung erstreckt. Der Ulnaschaft (10) verjüngt sich in eine vom Schwenklager (4) abgewandte Richtung auf sein unteres Ende (53) stetig. Der Ulnaschaft (10) ist lang und schlank ausgebildet. Der gesamte Ulnateil (2) besitzt eine gesamte Länge, die sich von seinem unteren Ende (53) bis zu seinem diesem gegenüberliegenden oberen Ende (52) erstreckt, von etwa 140 mm.

Quer zur Schwenkebene (51) verläuft eine Querebene (54). In dieser Querebene (54) ist jeweils eine Hinterkante (55) des Humerusteils (1) und eine Hinterkante (56) des Ulnateils (2) angeordnet. Die Hinterkante (55) begrenzt den Humerusteil (1) auf seiner hinteren Seite (57), während die Hinterkante (56) den Ulnateil (2) auf seiner hinteren Seite (57) begrenzt, die einem Schwenkwinkel (58) gegenüber liegt, der von den beiden Schäften (10, 36) im verschwenkten Zustand der Endeoprothese eingeschlossen wird.

Im gestreckten Zustand der Endoprothese erstreckt sich eine hintere Begrenzung (59) des Ulnaschaftes (10) entlang der Hinterkante (56). Darüber hinaus erstreckt sich in einem geringen Abstand (61) parallel zu der sich durch die Hinterkante (55, 56) erstreckenden Querebene (54) eine der hinteren Seite (57) zugewandte hintere Begrenzung (60) des Humerusschaftes (36). Dieser geringe Abstand (61) beträgt etwa 3 bis 5 mm.

Durch den Humerusschaft (36) erstreckt sich eine Längsachse (19), die im gestreckten Zustand der Endoprothese parallel zur Querebene (54) verläuft. Zwischen der Längsachse (19) und der sich durch die Hinterkante (55, 56) erstreckenden Querebene (54) ist ein geringer Abstand (62) vorgesehen, der etwa 5 bis 8 mm beträgt.

Durch das Schwenklager (4) erstreckt sich eine Mittellinie (64) quer zur Schwenkebene (51). Diese Mittellinie besitzt einen Abstand (63) von der Hinterkante (55), der größer als der zwischen der hinteren Begrenzung (60) und der Längsachse (19) liegende Abstand (62) ist. Dieser Abstand (63) zwischen der Mittellinie (64) und der Hinterkante (55) beträgt etwa 12 bis 16 mm.

Der Humerusschaft (36) besitzt gegenüber der Schwenkebene (51) eine seitliche Neigung (65). Diese Neigung entspricht einer Valgusstellung, die überlicherweise ein Humerusknochen gegenüber einem Ulnaknochen besitzt. Diese Neigung (65) des Humerusschaftes (36) beträgt etwa 10 bis 12° gegenüber der Schwenkebene (51).

Der Ulnaschaft besitzt eine Ulnalängsachse (9), die sich in Längsrichtung durch den Ulnaschaft (10) erstreckt. Diese Ulnalängsachse ist in einer quer zur Schwenkebene (51) verlaufenden Richtung um einen Übergang (20) gegenüber der Humeruslängsachse (19) versetzt. Dieser Übergang (20) erstreckt sich in eine der Neigung (65) abgewandte Richtung. Darüber hinaus ist der Übergang (20) bezüglich einer mittig durch das Schwenklager (4) verlaufenden Schwenkebene (51) gegenüber einem Quersteg (8) angeordnet, der sich quer zur Schwenkebene (51) erstreckt und am Ulnateil (2) befestigt ist. An diesem Quersteg (8) ist eine Pfanne (6) befestigt, die sich in eine vom Humerusteil (1) abgewandte Richtung öffnet und zur Aufnahme eines Radiuskopfes (7) vorgesehen ist, der den Radiusteil (3) an seinem dem Humerusteil (1) zugewandten oberen Ende begrenzt. Die Pfanne (6) besitzt eine Pfannenmittellinie (12), die quer zu einer sich durch das Schwenklager erstreckenden Schwenkebene (51) ebenso weit von der Schwenkebene (51) entfernt ist, wie die Ulnalängsachse (9). Diese ist gegenüber der Schwenkebene (51) um etwa 5 bis 8 mm versetzt. Bezüglich der Ulnalängsachse (9) ist der Quersteg (8) in gleicher Höhe am Ulnateil (1) befestigt wie der Übergang (20).

Der Übergang (20) mündet auf seiner dem Ulnaschaft (10) abgewandten Seite unmittelbar in einen Ulnalagerteil (66) des Schwenklagers (4) ein. Das Ulnalagerteil (66) ist als ein Halterungselement (18) ausgebildet das um eine im Humerusteil (1) befestigte Welle (32) schwenkbar gelagert ist. Das Halterungselement (18) kann als ein Haken (67) ausgebildet sein, der mit zwei Seitenschenkeln (21, 22) eine Ausnehmung (49) umschließt, die die Welle (32) übergreift. Die Welle (32) erstreckt sich quer zur Schwenkebene (51) durch ein im Humerusteil (1) ausgebildetes Gehäuse (37), in dem das Halterungselement (18) verschwenkbar angeordnet ist. Die Seitenschenkel (21, 22) erstrecken sich in Richtung der Schwenkebene (51) und verlaufen einander etwa parallel quer zur Hinterkante (56). Zwischen den beiden Seitenschenkeln (21, 22) ist eine als Hakenöffnung ausgebildete Ausnehmung (49) vorgesehen, deren Querschnitt etwa demjenigen der Welle (32) entspricht. Das Halterungselement (18) besitzt quer zur Schwenkebene (51) einen rechteckigen Querschnitt (68). Diesem Querschnitt (68) ist ein vom Gehäuse (37) umschlossener Innenraum (38) angepaßt. Dieser Innenraum (38) wird von zwei einander gegenüberliegenden Seitenwandungen (39, 40) begrenzt, die einander etwa planparallel und parallel zur Schwenkebene (51) verlaufen. An den Seitenwandungen (39, 40) ist die Welle (32) im Abstand (63) von der Hinterkante (55) befestigt. Die Größe dieses Abstandes (63) entspricht in etwa der Breite des Humerusteils (1) an seiner Einmündung an das Gehäuse (37).

Die Seitenwandungen (39, 40) sind auf ihren den Innenraum (38) zugewandten Innenseiten (69, 70) mit Gleitbelägen (42, 43) belegt, auf denen das Halterungselement (18) mit seinen Seitenbegrenzungen gleitend gelagert ist. Die Gleitbeläge (42, 43) bestehen zweckmäßigerweise aus Polyäthylen.

Der Innenraum (38) ist in Richtung auf den Ulnateil (2) nach unten offen und weist in Richtung auf den Humerusschaft (36) eine Gehäusedecke (41) auf, auf der ein Anschlag (44) für eine ihr zugewandte Oberkante (73) des Halterungselementes (18) ausgebildet ist. Dieser Anschlag (44) weist von der Gehäusedecke (41) in Richtung auf den Innenraum (38).

Die Seitenwandungen (39, 40) sind mindestens an ihren, dem Ulnateil (2) zugewandten unteren Ende (74, 75) mit bogenförmigen Begrenzungen (76) versehen. Diese bogenförmige Begrenzung (76) kann als ein Kreisbogen ausgebildet sein, dessen Mittelpunkt auf der Mittellinie (64) der Welle (32) liegt. Die Begrenzung (76) verläuft in Richtung auf den Ulnateil (2) oberhalb des Quersteges (8).

An den unteren Enden (74, 75) ist an jeder der Seitenwandungen (39, 40) jeweils ein vom Gehäuse (37) wegweisender Stützflügel (47, 48) befestigt, die sich entlang der unteren Begrenzung (76) erstrecken. Diese Stützflügel (47, 48) besitzen eine Breite, die etwa derjenigen des Innenraumes (38) entspricht. Jeder dieser Stützflügel weist eine dem Ulnateil (2) zugewandte untere Auflagefläche (77, 78) auf, die quer zur Längsrichtung der Stützflügel (47, 48) eine in Richtung auf den Ulnateil (2) weisende leichte Wölbung (79) aufweist. Die Stützflügel (47, 48) erstrecken sich auf einem Bogen von etwa 180° beginnend von der Hinterkante (55) entlang der Begrenzung (76).

Die Welle (32) ist mit einem Gleitbelag (80) ummantelt, auf dem der Ulnateil (2) mit der Ausnehmung (49) verschwenkbar gelagert ist. Diese Ausnehmung (49) befindet sich in einem Abstand (81) von der Hinterkante (56), dessen Größe etwa dem Abstand (63) entspricht. Dieser Abstand (81) ist im Bereich eines Basisschenkels (23) vorgesehen, der die beiden Seitenschenkel (21, 22) miteinander verbindet. Ein Herausgleiten der Welle (32) aus der Ausnehmung (49) wird mit Hilfe einer Sicherungsschraube (26) verhindert, die sich in etwa parallel zur Hinterkante (56) quer zur Richtung der Welle (32) vom oberen Seitenschenkel (21) in den unteren Seitenschenkel (22) erstreckt und mit ihrem Mittelteil (82) die Ausnehmung (49) verriegelt. Zweckmäßigerweise verläuft die Sicherungsschraube (26) im Bereich der sich mittig durch das Schwenklager (4) erstreckenden Schwenkebene (51).

Die Sicherungsschraube (26) erstreckt sich durch eine zylindrische Ausnehmung (24), die sich parallel zur Hinterkante (56) durch den oberen Seitenschenkel (21) an einer Stelle erstreckt, an der sie mit ihrem Mittelteil (82) die Welle (32) beaufschlagen kann, wenn diese sich durch die Ausnehmung (49) erstreckt. Zu diesem Zwecke ist in der Ausnehmung (24) ein Innengewinde (25) vorgesehen, durch das die Sicherungsschraube (26) in Richtung auf den unteren Seitenschenkel (22) verschraubt wird. Dabei greift ein auf der Sicherungsschraube (26) vorgesehenes Außengewinde (27) in das Innengewinde der Ausnehmung (24) hinein. Die Sicherungsschraube (26) wird mit ihrem dem oberen Seitenschenkel (21) abgewandten unteren Ende (83) in einem Sackloch (28) geführt, in das die Sicherungsschraube (26) hineinragt, wenn sie in vollem Umfange in die Ausnehmung (24) hineingeschraubt ist.

Im Bereich der Sicherungsschraube (26) ist in der Welle (32) eine Nut (50) vorgesehen. Die Sicherungsschraube (26) beaufschlagt die Welle (32) auf einem in die Welle (32) hineinragenden Boden (84) der Nut (50). Es ist aber auch möglich, der Nut (50) im Bereich des Bodens (84) eine kantige Ausbildung (85) zu verleihen und diese in einer quer zur Wellenlängsachse verlaufenden Schnittebene im wesentlichen als Quadrat oder Vieleck auszubilden. Bei einer derartigen Ausbildung der Nut (50) wird die Sicherungsschraube (26) durch eine sich bereichsweise parallel zu ihr erstreckende Anschlagfläche (86) der Nut (50) beaufschlagt. In diesem Falle muß jedoch die Welle (32) in den Seitenwandungen (39, 40) schwenkbar gelagert sein, so daß sie als eine Achse ausgebildet ist.

Es ist jedoch auch möglich, das Halterungselement (18) im wesentlichen als einen Zapfen (29) auszubilden, der eine sich im wesentlichen senkrecht zur Humeruslängsachse (19) erstreckende Bohrung (30) aufweist. Der Zapfen (29) ist von einer zylindrischen Ausnehmung (31) aufgenommen, die im Bereich der Welle (32) angeordnet ist. Die Welle (32) weist eine Bohrung (33) auf, die mit einem Innengewinde (34) versehen ist. In das Innengewinde (34) greift eine Madenschraube (35) ein. Die Madenschraube (35) durchdringt den Zapfen (29) im Bereich der Bohrung (30) und fixiert ihn gegenüber der Welle (32). Auch in diesem Falle muß die Welle (32) verschwenkbar in den Seitenwandungen (39, 40) als Achse gelagert sein.

Im gestreckten Zustand der Endoprothese verläuft eine sich durch den Radiusteil (3) erstreckende Radiusmittellinie (17) parallel zur Ulnalängsachse (9), wenn der Radiusteil (3) gegenüber dem Ulnateil (2) nicht verschwenkt ist. In diesem Falle beaufschlagt der Radiusteil (3) mit seinem Radiuskopf (7) die Pfanne (6) im Bereich der Pfannenmittellinie (12). Dabei wird der Radiuskopf (7) von der Radiusmittellinie (87) zentrisch durchdrungen. In eine von der Pfanne (6) abgewandte Richtung schließt sich an den Radiuskopf (7) entlang der Radiusmittellinie (17) der Radiusschaft (16) an. Dieser ist ebenso wie der Ulnaschaft (10) und der Humerusschaft (36) lang und schlank ausgebildet. Alle drei Schäfte (10, 16, 36) sind biegeelastisch ausgebildet, um sie möglichen Verformungen des sie aufnehmenden Knochens anpassen zu können.

In der Pfanne (6) weist der Radiuskopf (7) eine Lagerung (5) auf, die je nach einer Verdrehung des Radiusteils (3) gegenüber dem Ulnateil (2) mehr oder minder weit vom Ulnateil (2) enfernt sein kann. Je weiter der Radiusteil (3) gegenüber dem Ulnateil (2) bei einer Verdrehung des Unterarms verschwenkt ist, um so mehr wandert der Radiuskopf (7) vom Ulnateil (2) weg in eine äußere Lagerung (5). Zu diesem Zwecke weist der Radiuskopf (7) im wesentlichen eine kugelförmige Ausbildung auf. Die Pfanne (6) besitzt demgegenüber eine flach gewölbte Oberkante (87), entlang der der Radiuskopf (7) bei Verdrehbewegungen des Unterarmes frei wandern kann.

Der Radiuskopf kann aus einem Kunststoff, beispielsweise Polyäthylen ausgebildet sein. In diesen Falle besteht die Pfanne (6) im wesentlichen aus Metall. Es ist natürlich auch möglich, die Pfanne (6) mit einem Kunststoffüberzug zu versehen und den Radiuskopf (7) aus Metall auszubilden.

Der Radiuskopf (7) ist im Bereich seiner der Pfanne (6) abgewandten Ausdehnung mit einem Sockel (14) verbunden. Dieser leitet den Radiuskopf (7) in eine sich im wesentlichen parallel zum Quersteg (8) erstreckende Auflageplatte (15) über. Mit dieser liegt der Radiusteil (3) auf einem dem Radiuskopf (7) zugewandten oberen Ende des Radiusknochens auf. Im Bereich ihrer dem Sockel (14) abgewandten Begrenzung ist die Auflageplatte (15) mit dem Radiusschaft (16) verbunden, der sich entlang der Radiusmittellinie (17) in eine der Auflageplatte (15) abgewandten Richtung verjüngt.

Zur Implantation der Ellenbogengelenk-Endoprothese werden zunächst der Humerusteil (1), der Ulnateil (2) und der Radiusteil (3) in die diese aufnehmenden Knochen implantiert. Anschließend wird der Ulnateil (2) mit seinem Halterungselement (18) im Bereich des Gehäuses (37) aufgenommen. Diese Aufnahme erfolgt entweder durch ein Einhaken des Hakens (67) an der Welle (32) und eine anschließende Sicherung mit Hilfe der Sicherungsschraube (26) oder durch Einführen des als Zapfen (29) ausgebildeten Halterungselementes (18) in die Ausnehmung (31) und die anschließende Sicherung mit Hilfe der Madenschraube (35). Abschließend wird der Radiuskopf (7) in die Pfanne (6) eingeführt.

## Patentansprüche

1. Ellenbogengelenk-Endoprothese mit einem Humerusteil (1), um das in einem Schwenklager (4) verschwenkbar ein Ulnateil (2) gelagert ist, an dem eine als Pfanne (6) ausgebildete Lagerung (5) befestigt ist, in der ein Radiusteil (3) mit einem Radiuskopf (7) gelagert ist, dadurch gekennzeichnet, daß die Pfanne (6) eine derart größere flächenhafte Ausdehnung als der von ihr umschlossene Bereich des Radiuskopfes (7) aufweist, daß der Radiuskopf (7) im Bereich der Pfanne (6) sowohl in Richtung auf einen im Ulnateil (2) verlaufenden Ulnaschaft (10) als auch quer zu dieser Richtung beweglich ist.

2. Endoprothese nach Anspruch 1, dadurch gekennzeichnet, daß die Pfanne (6) im wesentlichen zentrisch zu einer Pfannenmittellinie (12) ausgebildet ist.

3. Endoprothese nach Anspruch 1 oder 2, dadurch gekennzeichnet, daß die Pfanne (6) dem Radiuskopf (7) angepaßt und diesen führend ausgebildet ist.

4. Endoprothese nach einem der Ansprüch 1 bis 3, dadurch gekennzeichnet, daß die Pfanne (6) im wesentlichen aus Metall ausgebildet ist.

5. Endoprothese nach einem der Ansprüche 1 bis 4, dadurch gekennzeichnet, daß der Radiuskopf (7) im wesentlichen aus Polyäthylen ausgebildet ist.

6. Endoprothese nach einem der Ansprüche 1 bis 5, dadurch gekennzeichnet, daß der Radiuskopf (7) im Bereich seiner der Pfanne (6) abgewandten Ausbildung in einen Sockel (14) einmündet.

7. Endoprothese nach Anspruch 6, dadurch gekennzeichnet, daß der Sockel (14) im Bereich seiner dem Radiuskopf (7) abgewandten Ausdehnung mit einer Auflageplatte (15) verbunden ist.

8. Endoprothese nach Anspruch 7, dadurch gekennzeichnet, daß die Auflageplatte (15) im Bereich ihrer dem Sockel (15) abgewandten Ausdehnung in einen Radiusschaft (16) einmündet.

9. Endoprothese nach Anspruch 8, dadurch gekennzeichnet, daß der Radiusschaft (16) sich in eine der Auflageplatte (15) abgewandte Richtung verjüngt.

10. Endoprothese nach einem der Ansprüche 8 oder 9, dadurch gekennzeichnet, daß der Radiusschaft (16) lang und schlank ausgebildet ist und eine Mittelachse aufweist, die den Radiuskopf (7) mittig durchmißt.

11. Endoprothese nach einem der Ansprüche 8 bis 10, dadurch gekennzeichnet, daß der Radiusschaft (16) etwa 50 mm lang ist.

12. Endoprothese nach einem der Ansprüche 8 bis 11, dadurch gekennzeichnet, daß der Radiusschaft (16) biegeelastisch ausgebildet ist.

13. Endoprothese nach Anspruch 8bis 12, dadurch gekennzeichnet, daß der Radiusschaft (16) aus Metall ausgebildet ist.

14. Endoprothese nach einem der Ansprüche 1 bis 13, dadurch gekennzeichnet, daß die Pfanne (6) im Bereich eines Quersteges (8) angeordnet ist, der sich im wesentlichen senkrecht zu einem am Ulnateil (2) ausgebildeten Ulnaschaft (10) und quer zu einer vom Schwenklager (4) vorgegebenen Schwenkebene (51) erstreckt.

15. Endoprothese nach einem der Ansprüche 1 bis 14, dadurch gekennzeichnet, daß das Schwenklager einen an einem dem Humerusteil (1) zugewandten oberen Ende (52) des Ulnateils (2) befestigten Ulnalagerteil (66) aufweist und ein zur Verankerung dienender Ulnaschaft (10) sich in eine vom oberen Ende (52) abgewandte Richtung in Richtung auf ein unteres Ende (53) erstreckt.

16. Endoprothese nach Anspruch 15, dadurch gekennzeichnet, daß der Ulnaschaft (10) sich in Richtung auf das untere Ende (53) stetig verjüngt.

17. Endoprothese nach einem der Ansprüche 15 oder 16, dadurch gekennzeichnet, daß der Ulnaschaft (10) lang und schlank ausgebildet ist und das gesamte Ulnateil (2) von seinem oberen bis zu seinem unteren Ende (53) eine Länge von etwa 140 mm aufweist.

18. Endoprothese nach einem der Ansprüche 14 bis 17, dadurch gekennzeichnet daß in einer quer zur Schwenkebene (51) verlaufenden Querebene (54) jeweils eine Hinterkante (55, 56) des Humerusteils (1) und des Ulnateils (2) angeordnet sind, von denen die eine den Humerusteil (1) und die andere den Ulnateil (2) jeweils auf ihrer hinteren Seite (57) begrenzt, die einem von den beiden Schäften (10, 36) im verschwenkten Zustand gebildeten Schwenkwinkel (58) gegenüberliegt.

19. Endoprothese nach Anspruch 18, dadurch gekennzeichnet, daß im gestreckten Zustand eine der hinteren Seite (57) zugewandte hintere Begrenzung (59) des Ulnaschaftes (10) sich entlang der Hinterkante (56) erstreckt.

20. Endoprothese nach einem der Ansprüche 18 oder 19, dadurch gekennzeichnet, daß im gestreckten Zustand eine der hinteren Seite (57) zugewandte hintere Begrenzung (60) des Humerusschaftes (36) in einem geringen Abstand (61) parallel zu der sich durch die Hinterkante (55, 56) erstreckende Querebene (54) verläuft.

21. Endoprothese nach Anspruch 20, dadurch gekennzeichnet, daß der geringe Abstand (61) etwa 3 bis 5 mm beträgt.

22. Endoprothese nach einem der Ansprüche 18 bis 21, dadurch gekennzeichnet, daß sich durch den Humerusschaft (36) eine Längsachse (19) erstreckt, die im gestreckten Zustand parallel zur Querebene (54) verläuft.

23. Endoprothese nach Anspruch 22, dadurch gekennzeichnet, daß zwischen der Längsachse (19) und der sich durch die Hinterkante (55, 56) erstreckenden Querebene (54) ein geringer Abstand (62) vorgesehen ist.

24. Endoprothese nach Anspruch 23, dadurch gekennzeichnet, daß der geringe Abstand (62) etwa 5 bis 8 mm beträgt.

25. Endoprothese nach einem der Ansprüche 14 bis 24, dadurch gekennzeichnet, daß eine sich durch das Schwenklager (4) erstrekkende Mittellinie (64) quer zur Schwenkebene (51) durch den Ulnateil (2) in einem Abstand (63) von der Hinterkante (55) verläuft, der größer als der zwischen der hinteren Begrenzung (60) und der Längsachse (19) liegende Abstand (62) ist.

26. Endoprothese nach Anspruch 25, dadurch gekennzeichnet, daß der Abstand (63) zwischen der Mittellinie (64) und der Hinterkante (55) etwa 12 bis 16 mm ist.

27. Endoprothese nach einem der Ansprüche 1 bis 26, dadurch gekennzeichnet, daß der Humerusschaft (36) gegenüber der Schwenkebene (51) eine seitliche Neigung (65) besitzt.

28. Endoprothese nach Anspruch 27, dadurch gekennzeichnet, daß die Neigung (65) einer Valgusstellung entspricht.

29. Endoprothese nach einem der Ansprüche 27 oder 28, dadurch gekennzeichnet, daß die Neigung (65) des Humerusschaftes (36) etwa 10 bis 12 ° gegenüber der Schwenkebene (51) beträgt.

30. Endoprothese nach einem der Ansprüche 1 bis 29, dadurch gekennzeichnet, daß eine sich durch den Ulnaschaft (10) erstrekkende Ulnalängsachse (9) in einer quer zur Schwenkebene (51) verlaufenden Richtung um einen Übergang (20) gegenüber der Humeruslängsachse (19) versetzt ist.

31. Endoprothese nach Anspruch 30, dadurch gekennzeichnet, daß der Übergang (20) sich in eine der Neigung (65) abgewandte Richtung erstreckt.

32. Endoprothese nach einem der Ansprüche 30 oder 31, dadurch gekennzeichnet, daß der Übergang (20) sich bezüglich einer mittig durch das Schwenklager (4) verlaufenden Schwenkebene (51) gegenüber dem Quersteg (8) erstreckt.

33. Endoprothese nach einem der Ansprüche 30 bis 32, dadurch gekennzeichnet, daß die um den Übergang (20) gegenüber der durch das Schwenklager (4) verlaufenden Schwenkachse (51) versetzte Ulnalängsachse (9) etwa ebenso weit von der Schwenkebene (51) entfernt ist wie die Pfannenmittellinie (12).

34. Endoprothese nach einem der Ansprüche 30 bis 33, dadurch gekennzeichnet, daß die Ulnalängsachse (9) gegenüber der Schwenkebene (51) um etwa 5 bis 8 mm versetzt ist.

35. Endoprothese nach einem der Ansprüche 30 bis 34, dadurch gekennzeichnet, daß bezüglich der Ulnalängsachse (9) der Quersteg (8) in gleicher Höhe wie der Übergang (20) vorgesehen ist.

36. Endoprothese nach Anspruch 30 bis 35, dadurch gekennzeichnet, daß der Übergang (20) auf seiner der Ulnalängsachse (9) abgewandten Seite unmittelbar in einen Ulnalagerteil (66) des Schwenklagers (4) einmündet.

37. Endoprothese nach einem der Ansprüche 15 bis 36, dadurch gekennzeichnet, daß der Ulnalagerteil (66) des Schwenklagers (4) als ein um eine im Humerusteil (1) befestigte Welle (32) schwenkbares Halterungselement (18) ausgebildet ist.

38. Endoprothese nach Anspruch 37, dadurch gekennzeichnet, daß das Halterungselement (18) als ein Haken (67) ausgebildet ist, der mit zwei Seitenschenkeln (21, 22) eine Ausnehmung (49) umschließt, die die Welle (32) übergreift.

39. Endoprothese nach einem der Ansprüche 37 oder 38, dadurch gekennzeichnet, daß die Welle (32) sich quer zur Schwenkebene (51) durch ein im Humerusteil (1) ausgebildetes Gehäuse (37) erstreckt, in dem das Halterungselement (18) verschwenkbar angeordnet ist.

40. Endoprothese nach einem der Ansprüche 38 oder 39, dadurch gekennzeichnet, daß die Seitenschenkel (21, 22) sich in Richtung der Schwenkebene (51) und einander etwa parallel verlaufend quer zur Hinterkante (56) erstrecken.

41. Endoprothese nach einem der Ansprüche 38 bis 40, dadurch gekennzeichnet, daß zwischen den beiden Seitenschenkeln (21, 22) eine als Hakenöffnung ausgebildete Ausnehmung (49) ausgebildet ist, deren Querschnitt etwa demjenigen der Welle (32) entspricht.

42. Endoprothese nach einem der Ansprüche 37 bis 41, dadurch gekennzeichnet, daß das Halterungselement (18) quer zur Schwenkebene (51) einen rechteckigen Querschnitt (68) aufweist und das Gehäuse (37) einen diesem rechteckigen Querschnitt (68) angepaßten Innenraum (38) aufweist.

43. Endoprothese nach Anspruch 42, dadurch gekennzeichnet, daß der Innenraum (38) von zwei einander gegenüberliegenden Seitenwandungen (39, 40) begrenzt ist, die einander etwa planparallel und parallel zur Schwenkebene (51) verlaufen.

44. Endoprothese nach Anspruch 43, dadurch gekennzeichnet, daß an den Seitenwandungen (39, 40) die Welle (32) im Abstand (63) von der Hinterkante (55) befestigt ist, dessen Größe in etwa der Breite des Humerusteils (1) an seiner Einmündung in das Gehäuse (37) entspricht.

45. Endoprothese nach einem der Ansprüche 43 oder 44, dadurch gekennzeichnet, daß die Seitenwandungen (39, 40) auf ihren dem Innenraum (38) zugewandten Innenseiten (69, 70) Gleitbeläge (42, 43) aufweisen, auf denen das Halterungselement (18) mit seinen Seitenbegrenzungen (71, 72) gleitend gelagert ist.

46. Endoprothese nach Anspruch 45, dadurch gekennzeichnet, daß die Gleitbeläge (42, 43) aus Polyäthylen bestehen.

47. Endoprothese nach einem der Ansprüche 42 bis 46, dadurch gekennzeichnet, daß der Innenraum (38) in Richtung auf den Ulnateil (2) nach unten offen ist und in Richtung auf den Humerusschaft (36) eine Gehäusedecke (41) aufweist, auf der ein Anschlag (44) für eine ihr zugewandte Oberkante (73) des Halterungselementes (18) ausgebildet ist.

48. Endoprothese nach einem der Ansprüche 43 bis 46, dadurch gekennzeichnet, daß die Seitenwandungen (39, 40) mindestens an ihren dem Ulnateil (2) zugewandten unteren Enden (74, 75) eine bogenförmige Begrenzung (76) aufweisen.

49. Endoprothese nach Anspruch 48, dadurch gekennzeichnet, daß die bogenförmige Begrenzung (76) als Kreisbogen ausgebildet ist, dessen Mittelpunkt auf der Mittellinie (64) der Welle (32) liegt.

50. Endoprothese nach einem der Ansprüche 43 bis 49, dadurch gekennzeichnet, daß die Begrenzung der Seitenwandungen in Richtung auf den Ulnateil (2) oberhalb des Quersteges (8) verläuft.

51. Endoprothese nach einem der Ansprüche 43 bis 50, dadurch gekennzeichnet, daß an jeder der Seitenwandungen (39, 40) jeweils ein vom Gehäuse (37) wegweisender Stützflügel (47, 48) befestigt ist, der sich entlang der unteren Begrenzung (76) erstreckt.

52. Endoprothese nach Anspruch 51, dadurch gekennzeichnet, daß die Stützflügel (47, 48) jeweils eine Breite besitzen, die etwa derjenigen des Innenraumes (38) entsprechend.

53. Endoprothese nach einem der Ansprüche 51 oder 52, dadurch gekennzeichnet, daß jeder der Stützflügel (47, 48) eine dem Ulnateil (2) zugewandte untere Auflagefläche (77, 78) aufweist, die quer zur Längsrichtung der Stützflügel (47, 48) eine in Richtung auf den Ulnateil (2) weisende leichte Wölbung (79) aufweist.

54. Endoprothese nach einem der Ansprüche 51 bis 53, dadurch gekennzeichnet, daß die Stützflügel (47, 48) sich auf einem Bogen von 180° beginnend von der Hinterkante (55) entlang der Begrenzung (76) erstrecken.

55. Endoprothese nach einem der Ansprüche 37 bis 54, dadurch gekennzeichnet, daß die Welle (32) mit einem Gleitbelag (80) versehen ist.

56. Endoprothese nach Anspruch 55, dadurch gekennzeichnet, daß der Gleitbelag aus Polyäthylen besteht.

57. Endoprothese nach Anspruch 37 bis 56, dadurch gekennzeichnet, daß die Halterung (18) auf der Welle (32) mit Hilfe einer Sicherungsschraube (26) geführt ist, die sich quer zur Richtung der Welle (32) von einem Schenkel (21) zum anderen Schenkel (22) erstreckt und die Welle (32) auf ihrer Oberfläche beaufschlagt.

58. Endoprothese nach Anspruch 57, dadurch gekennzeichnet, daß die Sicherungsschraube (26) sich durch eine Nut (50) erstreckt, die in eine der Halterung (18) zugewandte Oberfläche der Welle (32) eingearbeitet ist und die Sicherungsschraube (26) auf mindestens einem Teil ihrer Oberfläche umschließt.

59. Endoprothese nach Anspruch 58, dadurch gekennzeichnet, daß die Nut (50) einen eckigen Querschnitt der Welle (32) begrenzt und die Sicherungsschraube (26) sich entlang einer Seitenkante des eckigen Querschnittes erstreckt.

60. Endoprothese nach einem der Ansprüche 43 bis 59, dadurch gekennzeichnet, daß die Halterung (18) durch eine sich durch die Welle (32) erstreckende Bohrung hindurchragt und in dieser befestigt ist und daß die Welle (32) in den Seitenwandungen (39, 40) verschwenkbar gelagert ist.

61. Endoprothese nach einem der Ansprüche 2 bis 60, dadurch gekennzeichnet, daß die Radiusmittellinie (17) sich entlang der Pfannenmittellinie (12) erstreckt und im unverdrehten Zustand des Unterarmes parallel zum Ulnaschaft verläuft.

62. Endoprothese nach einem der Ansprüche 1 bis 61, dadurch gekennzeichnet, daß der Radiuskopf (7) gleitbar innerhalb der Pfanne (6) gelagert ist.

63. Endoprothese nach einem der Ansprüche 1 bis 62, dadurch gekennzeichnet, daß die Pfanne (6) bezüglich des Radiuskopfes (7) flach gewölbt ist.

## Claims

1. An elbow joint endoprosthesis comprising a humerus member (1) about which there is mounted pivotably about a pivot mounting (4) an ulna member (2) to which there is fixed a mounting means (5) which is in the form of a socket (6) and in which a radius member (3) is mounted with a radius head (7), characterised in that the socket (6) is of a surface-area extent which is so much greater than the region of the radius head (7), which is embraced thereby, that the radius head (7) is movable in the region of the socket (6) both in a direction towards an ulna shank (10) extending in the ulna member (2), and also transversely relative to said direction.

2. An endoprosthesis according to claim 1 characterised in that the socket (6) is formed substantially centrally relative to a socket centre line (12).

3. An endoprosthesis according to claim 1 or claim 2 characterised in that the socket (6) is adapted to the radius head (7) and is designed to guide same.

4. An endoprosthesis according to one of claims 1 to 3 characterised in that the socket (6) is substantially formed from metal.

5. An endoprosthesis according to one of claims 1 to 4 characterised in that the radius head (7) is substantially formed from polyethylene.

6. An endoprosthesis according to one of claims 1 to 5 characterised in that in the region of its configuration which faces away from the socket (6) the radius head (7) connects to a support portion (14).

7. An endoprosthesis according to claim 6 characterised in that in the region of its extent which faces away from the radius head (7) the support portion (14) is connected to a contact plate (15).

8. An endoprosthesis according to claim 7 characterised in that in the region of its extent which faces away from the support portion (14) the contact plate (15) connects to a radius shank (16).

9. An endoprosthesis according to claim 8 characterised in that the radius shank (16) tapers in a direction away from the contact plate (15).

10. An endoprosthesis according to one of claims 8 and 9 characterised in that the radius shank (16) is of a long and slender configuration and has a central axis which passes centrally through the radius head (7).

11. An endoprosthesis according to one of claims 8 to 10 characterised in that the radius shank (16) is about 50 mm in length.

12. An endoprosthesis according to one of claims 8 to 11 characterised in that the radius shank (16) is of a flexurally resilient nature.

13. An endoprosthesis according to claims 8 to 12 characterised in that the radius shank (16) is made from metal.

14. An endoprosthesis according to one of claims 1 to 13 characterised in that the socket (6) is arranged in the region of a transverse limb (8) which extends substantially perpendicularly to an ulna shank (10) formed on the ulna member (2) and transversely to a pivot plane (51) which is predetermined by the pivot mounting (4).

15. An endoprosthesis according to one of claims 1 to 14 characterised in that the pivot mounting has an ulna mounting portion (66) fixed to an upper end (52) of the ulna member (2), that is towards the humerus member (1), and an ulna shank (10) which serves for anchoring purposes extends in a direction which is away from the upper end (52), towards a lower end (53).

16. An endoprosthesis according to claim 15 characterised in that the ulna shank (10) progressively tapers towards the lower end (53).

17. An endoprosthesis according to one of claims 15 and 16 characterised in that the ulna shank (10) is of a long and slender configuration and the entire ulna member (2) is of a length of about 140 mm from its upper end to its lower end (53).

18. An endoprosthesis according to one of claims 14 to 17 characterised in that arranged in a transverse plane (54) which extends transversely relative to the pivot plane (51) are a rear edge (55, 56) of the humerus member (1) and the ulna member (2) respectively, of which the one edge respectively delimits the humerus member (1) and the other the ulna member (2) on its rear side (57) which is disposed opposite a pivot angle (58) which is formed by the two shanks (10, 36) in the pivoted condition.

19. An endoprosthesis according to claim 18 characterised in that in the extended condition a rear boundary (59) of the ulna shank (10), which is towards the rear side (57), extends along the rear edge (56).

20. An endoprosthesis according to one of claims 18 and 19 characterised in that in the extended condition a rear boundary (60) of the humerus shank (36), which is towards the rear side (57), extends at a small spacing (61) and parallel relative to the transverse plane (54) which extends through the rear edge (55, 56).

21. An endoprosthesis according to claim 20 characterised in that the small spacing (61) is about 3 to 5 mm.

22. An endoprosthesis according to one of claims 18 to 21 characterised in that extending through the humerus shank (36) is a longitudinal axis (19) which in the extended condition extends parallel to the transverse plane (54).

23. An endoprosthesis according to claim 22 characterised in that there is a spell spacing (62) between the longitudinal axis (19) and the transverse plane (54) which extends through the rear edge (55, 56).

24. An endoprosthesis according to claim 23 characterised in that the small spacing (62) is about 5 to 8 mm.

25. An endoprosthesis according to one of claims 14 to 24 characterised in that a centre line (64) which extends through the pivot mounting (4) passes transversely to the pivot plane (51) through the ulna member (2) at a spacing (63) from the rear edge (55), which is larger than the spacing (62) between the rear boundary (60) and the longitudinal axis (19).

26. An endoprosthesis according to claim 25 characterised in that the spacing (63) between the centre line (64) and the rear edge (55) is about 12 to 16 mm.

27. An endoprosthesis according to one of claims 1 to 26 characterised in that the humerus shank (36) has a lateral inclination (65) relative to the pivot plane (51).

28. An endoprosthesis according to claim 27 characterised in that the inclination (65) corresponds to a valgus position.

29. An endoprosthesis according to one of claims 27 and 28 characterised in that the inclination (65) of the humerus shank (36) is about 10 to 12° relative to the pivot plane (51).

30. An endoprosthesis according to one of claims 1 to 29 characterised in that an ulna longitudinal axis (9) which extends through the ulna shank (10) is displaced relative to the humerus longitudinal axis (19) by a transition portion (20) in a direction extending transversely to the pivot plane (51).

31. An endoprosthesis according to claim 30 characterised in that the transition portion (20) extends in a direction which is away from the inclination (65).

32. An endoprosthesis according to one of claims 30 and 31 characterised in that the transition portion (20) extends opposite the transverse limb (8) with respect to a pivot plane (51) extending centrally through the pivot mounting (4).

33. An endoprosthesis according to one of claims 30 to 32 characterised in that the ulna longitudinal axis (9) which is displaced by the transition portion (20) relative to the pivot axis (51) which extends through the pivot mounting (4) is approximately as far away from the pivot plane (51) as the socket centre line (12).

34. An endoprosthesis according to one of claims 30 to 33 characterised in that the ulna longitudinal axis (9) is displaced by about 5 to 8 mm relative to the pivot plane (51).

35. An endoprosthesis according to one of claims 30 to 34 characterised in that the transverse limb (8) is disposed at the same level as the transition portion (20) with respect to the ulna longitudinal axis (9).

36. An endoprosthesis according to claims 30 to 35 characterised in that on its side which is remote from the ulna longitudinal axis (9) the transition portion (20) connects directly to an ulna mounting portion (66) of the pivot mounting (4).

37. An endoprosthesis according to one of claims 15 to 36 characterised in that the ulna mounting portion (66) of the pivot mounting (4) is in the form of a holding element (18) which is pivotable about a shaft (32) fixed in the humerus member (1).

38. An endoprosthesis according to claim 37 characterised in that the holding element (18) is in the form of a hook (67) which with two side legs (21, 22) embraces an opening (49) which engages over the shaft (32).

39. An endoprosthesis according to one of claims 37 and 38 characterised in that the shaft (32) extends transversely to the pivot plane (51) through a housing (37) which is provided in the humerus member (1) and in which the holding element (18) is pivotably arranged.

40. An endoprosthesis according to one of claims 38 and 39 characterised in that the side legs (21, 22) extend in the direction of the pivot plane (51) and in approximately mutually parallel relationship transversely with respect to the rear edge (56).

41. An endoprosthesis according to one of claims 38 to 40 characterised in that provided between the two side legs (21, 22) is an opening (49) which is in the form of a hook opening and the cross-section of which approximately corresponds to that of the shaft (32).

42. An endoprosthesis according to one of claims 37 to 41 characterised in that transversely with respect to the pivot plane (51) the holding element (18) is of a rectangular cross-section (68) and the housing (37) has an internal space (38) which is adapted to said rectangular cross-section (68).

43. An endoprosthesis according to claim 42 characterised in that the internal space (38) is defined by two mutually oppositely disposed side walls (39, 40) which extend in approximately plane-parallel relationship with each other and parallel to the pivot plane (51).

44. An endoprosthesis according to claim 43 characterised in that the shaft (32) is fixed to the side walls (39) at the spacing (63) from the rear edge (55), the magnitude of which approximately corresponds to the width of the humerus member (1) at the location at which it connects to the housing (37).

45. An endoprosthesis according to one of claims 43 and 44 characterised in that on their insides (69, 70) which are towards the internal space (38) the side walls (39, 40) have anti-friction coatings (42, 43) on which the holding element (18) is slidably mounted with its side boundaries (71, 72).

46. An endoprosthesis according to claim 45 characterised in that the anti-friction coatings (42, 43) comprise polyethylene.

47. An endoprosthesis according to one of claims 42 to 46 characterised in that the internal space (38) is downwardly open in a direction towards the ulna member (2) and in a direction towards the humerus shank (36) has a housing cover (41) on which there is provided an abutment (44) for an upper edge (73), which is towards the housing cover, of the holding element (18).

48. An endoprosthesis according to one of claims 43 to 46 characterised in that the side walls (39, 40) have an arcuate boundary (76) at least at their lower ends (74, 75) which are towards the ulna member (2).

49. An endoprosthesis according to claim 48 characterised in that the arcuate boundary (76) is in the form of a circular arc whose centre point is on the centre line (64) of the shaft (32).

50. An endoprosthesis according to one of claims 43 to 49 characterised in that the boundary of the side walls extends in a direction towards the ulna member (2) above the transverse limb (8).

51. An endoprosthesis according to one of claims 43 to 50 characterised in that secured to each of the side walls (39, 40) is a respective suport wing (47, 48) which faces away from the housing (37) and which extends along the lower boundary (76).

52. An endoprosthesis according to claim 51 characterised in that the support wings (47, 48) are each of a width which approximately corresponds to that of the internal space (38).

53. An endoprosthesis according to one of claims 51 and 52 characterised in that each of the support wings (47, 48) has a lower contact surface (77, 78) which is towards the ulna member (2) and which, transversely to the longitudinal direction of the support wings (47, 48), has a slight curvature (79) facing in a direction towards the ulna member (2).

54. An endoprosthesis according to one of claims 51 to 53 characterised in that the support wings (47, 48) extend on an arc of 180°, beginning from the rear edge (55), along the boundary (76).

55. An endoprosthesis according to one of claims 37 to 54 characterised in that the shaft (32) is provided with an anti-friction coating (80).

56. An endoprosthesis according to claim 55 characterised in that the anti-friction coating comprises polyethylene.

57. An endoprosthesis according to claims 37 to 56 characterised in that the holding element (18) is guided on the shaft (32) by means of a securing screw (26) which extends transversely to the direction of the shaft (32) from one leg (21) to the other leg (22) and which acts on the shaft (32) on the surface thereof.

58. An endoprosthesis according to claim 57 characterised in that the securing screw (26) extends through a groove (50) which is provided in a surface of the shaft (32), that is towards the holding element (18), and embraces the securing screw (26) over at least a part of its surface.

59. An endoprosthesis according to claim 58 characterised in that the groove (50) delimits an angular cross-section of the shaft (32) and the securing screw (26) extends along a side edge of the angular cross-section.

60. An endoprosthesis according to one of claims 43 to 59 characterised in that the holding element (18) projects through a bore extending through the shaft (32) and is fixed in the bore and that the shaft (32) is mounted pivotably in the side walls (39, 40).

61. An endoprosthesis according to one of claims 2 to 60 characterised in that the radius centre line (17) extends along the socket centre line (12) and in the non-twisted condition of the forearm extends parallel to the ulna shank.

62. An endoprosthesis according to one of claims 1 to 61 characterised in that the radius head (7) is mounted slidably within the socket (6).

63. An endoprosthesis according to one of claims 1 to 62 characterised in that the socket (6) is of a shallow curvature with respect to the radius head (7).

## Revendications

1. Endoprothèse de l'articulation du coude comprenant un humérus (1), autour duquel peut pivoter un cubitus (2) monté dans un palier pivotant (4), contre lequel est fixé un palier (5) en forme de calotte (6), dans laquelle est logé un radius (3) comprenant une tête de radius (7), caractérisée en ce que la calotte (6) forme une cavité de surface plus grande que la zone de la tête du radius (7) qu'elle englobe, de telle sorte que la tête du radius (7) dans la zone de la calotte (6) puisse se déplacer non seulement dans le sens d'une tige ulnaire (10) qui s'étend dans le cubitus (2), mais aussi transversalement par rapport à cette direction.

2. Endoprothèse selon la revendication 1, caractérisée en ce que la calotte (6) est centrée pour l'essentiel par rapport à une ligne médiane de la calotte (12).

3. Endoprothèse selon la revendication 1 ou 2, caractérisée en ce que la calotte (6) est adaptée à la tête du radius (7) et formée pour guider cette dernière.

4. Endoprothèse selon l'une quelconque des revendications 1 à 3, caractérisée en ce que la calotte (6) est essentiellement métallique.

5. Endoprothèse selon l'une quelconque des revendications 1 à 4, caractérisée en ce que la tête du radius (7) est essentiellement formée en polyéthylène.

6. Endoprothèse selon l'une quelconque des revendications 1 à 5, caractérisée en ce que la tête du radius (7) s'engage dans un socle (14), dans la partie opposée à la calotte (6).

7. Endoprothèse selon la revendication 6, caractérisée en ce que le socle (14) est relié à une plaque de support (15), dans la partie opposée à la tête du radius (7).

8. Endoprothèse selon la revendication 7, caractérisée en ce que la plaque de support (15) s'engage dans une tige du radius (16), dans la partie opposée au socle (15).

9. Endoprothèse selon la revendication 8, caractérisée en ce que la tige du radius (16) se rétrécit dans le prolongement opposé à la plaque de support (15).

10. Endoprothèse selon la revendication 8 ou 9, caractérisée en ce que la tige du radius (16), longue et mince, est formée d'un axe médian qui traverse le milieu de la tête du radius (7).

11. Endoprothèse selon l'une quelconque des revendications 8 à 10, caractérisée en ce que la tige du radius (16) mesure environ 50 mm de long.

12. Endoprothèse selon l'une quelconque des revendications 8 à 11, caractérisée en ce que la tige du radius (16) est une tige souple.

13. Endoprothèse selon l'une quelconque des revendications 8 à 12, caractérisée en ce que la tige du radius (16) est une tige métallique.

14. Endoprothèse selon l'une quelconque des revendications 1 à 13, caractérisée en ce que la calotte (6) est disposée dans la zone d'une traverse transversale (8), laquelle est essentiellement perpendiculaire à une tige ulnaire (10) formée sur le cubitus (2) et s'étend dans le sens transversal par rapport à un plan de pivotement (51) défini par le palier pivotant (4).

15. Endoprothèse selon l'une quelconque des revendications 1 à 14, caractérisée en ce que le palier pivotant est formé d'un palier ulnaire (66), fixé contre l'extrémité supérieure (52) du cubitus (2) orientée vers l'humérus (1), et une tige ulnaire (10) servant d'ancrage s'étend dans le sens opposé à l'extrémité supérieure (52) en direction d'une extrémité inférieure (53).

16. Endoprothèse selon la revendication 15, caractérisée en ce que la tige ulnaire (10) se rétrécit progressivement dans le sens de l'extrémité inférieure (53).

17. Endoprothèse selon la revendication 15 ou 16, caractérisée en ce que la tige ulnaire (10) est longue et mince et l'ensemble du cubitus (2), de l'extrémité supérieure jusqu'à l'extrémité inférieure (53), mesure environ 140 mm de long.

18. Endoprothèse selon l'une quelconque des revendications 14 à 17, caractérisée en ce que, dans un plan transversal (54), qui s'étend transversalement par rapport au plan de pivotement (51), sont disposées les arêtes arrières (55, 56) respectives de l'humérus (1) et du cubitus (2), dont l'une limite l'humérus (1) et l'autre le cubitus (2) par la face arrière (57), qui est placée face à un angle de pivotement (58) formé par les deux tiges (10, 36), en position de pivotement.

19. Endoprothèse selon la revendication 18, caractérisée en ce que, en position étirée, une zone limite arrière (59) de la tige ulnaire (10), orientée vers la face arrière (57), se prolonge le long de l'arête arrière (56).

20. Endoprothèse selon la revendication 18 ou 19, caractérisée en ce que, en position étirée, une zone limite arrière (60) de la tige de l'humérus (36), orientée vers la face arrière (57), s'étend avec un faible intervalle (61) parallèlement au plan transversal (54) qui s'étend à travers l'arête arrière (55, 56).

21. Endoprothèse selon la revendication 20, caractérisée en ce que le faible intervalle (61) mesure environ 3 à 5 mm.

22. Endoprothèse selon l'une quelconque des revendications 18 à 21, caractérisée en ce qu'un axe longitudinal (19) s'étend dans la tige de l'humérus (36), lequel est parallèle au plan transversal (54) en position étirée.

23. Endoprothèse selon la revendication 22, caractérisée en ce qu'un faible intervalle (62) est prévu entre l'axe longitudinal (19) et le plan transversal (54) qui s'étend à travers l'arête arrière (55, 56).

24. Endoprothèse selon la revendication 23, caractérisée en ce que le faible intervalle (62) mesure environ 5 à 8 mm.

25. Endoprothèse selon l'une quelconque des revendications 14 à 24, caractérisée en ce qu'une ligne médiane (64), qui s'étend à travers le palier pivotant (4), se prolonge transversalement par rapport au plan de pivotement (51) à travers le cubitus (2) à un intervalle déterminé (63) de l'arête arrière (55), lequel est supérieur à l'intervalle (62) situé entre la zone limite arrière (60) et l'axe longitudinal (19).

26. Endoprothèse selon la revendication 25, caractérisée en ce que l'intervalle (63) entre la ligne médiane (64) et l'arête arrière (55) mesure environ 12 à 16 mm.

27. Endoprothèse selon l'une quelconque des revendications 1 à 26, caractérisée en ce que la tige de l'humérus (36) forme une inclinaison latérale (65) par rapport au plan de pivotement (51).

28. Endoprothèse selon la revendication 27, caractérisée en ce que l'inclinaison (65) correspond à une position valga.

29. Endoprothèse selon la revendication 27 ou 28, caractérisée en ce que l'inclinaison (65) de la tige de l'humérus (36) forme un angle de 10 à 12° environ par rapport au plan de pivotement (51).

30. Endoprothèse selon l'une quelconque des revendications 1 à 29, caractérisée en ce qu'un axe longitudinal ulnaire (9), qui s'étend à travers la tige ulnaire (10), est décalé dans le sens transversal par rapport au plan de pivotement (51) de la valeur d'un passage (20) par rapport à l'axe longitudinal de l'humérus (19).

31. Endoprothèse selon la revendication 30, caractérisée en ce que le passage (20) s'étend dans le sens opposé à l'inclinaison (65).

32. Endoprothèse selon la revendication 30 ou 31, caractérisée en ce que le passage (20) s'étend face à la traverse transversale (8) par rapport à un plan de pivotement (51) qui s'étend par le milieu du palier pivotant (4).

33. Endoprothèse selon l'une quelconque des revendications 30 à 32, caractérisée en ce que l'axe longitudinal ulnaire (9), décalé de la valeur du passage (20) par rapport à l'axe de pivotement (51) qui s'étend à travers le palier pivotant (4), est situé pratiquement à égale distance du plan de pivotement (51) et de la ligne médiane de la calotte (12).

34. Endoprothèse selon l'une quelconque des revendications 30 à 33, caractérisée en ce que l'axe longitudinal ulnaire (9) est décalé d'environ 5 à 8 mm par rapport au plan de pivotement (51).

35. Endoprothèse selon l'une quelconque des revendications 30 à 34, caractérisée en ce que, par rapport à l'axe longitudinal ulnaire (9), la traverse transversale (8) est disposée à même hauteur que le passage (20).

36. Endoprothèse selon l'une quelconque des revendications 30 à 35, caractérisée en ce que la face du passage (20), opposée à l'axe longitudinal ulnaire (9), s'engage directement dans un palier ulnaire (66) du palier pivotant (4).

37. Endoprothèse selon l'une quelconque des revendications 15 à 36, caractérisée en ce que le palier ulnaire (66) du palier pivotant (4) constitue un élément de maintien (18) qui peut pivoter autour d'un arbre (32) fixé dans l'humérus (1).

38. Endoprothèse selon la revendication 37, caractérisée en ce que l'élément de maintien (18) forme un crochet (67) dont les deux branches latérales (21, 22) englobent un évidement (49) qui s'engage au-dessus de l'arbre (32).

39. Endoprothèse selon la revendication 37 ou 38, caractérisée en ce que l'arbre (32) s'étend transversalement par rapport au plan de pivotement (51) à travers un boîtier (37) formé dans l'humérus (1), dans lequel est logé l'élément de maintien (18) de manière à pouvoir pivoter.

40. Endoprothèse selon la revendication 38 ou 39, caractérisée en ce que les branches latérales (21, 22), pratiquement parallèles l'une à l'autre, s'étendent dans le sens du plan de pivotement (51) et transversalement par rapport à l'arête arrière (56).

41. Endoprothèse selon l'une quelconque des revendications 38 à 40, caractérisée en ce qu'un évidement (49) constituant l'ouverture du crochet est formé entre les deux branches latérales (21, 22), la section dudit évidement correspondant pratiquement à celle de l'arbre (32).

42. Endoprothèse selon l'une quelconque des revendications 37 à 41, caractérisée en ce que l'élément de maintien (18) présente une section rectangulaire (68) transversale par rapport au plan de pivotement (51) et le boitier (37) présente un espace intérieur (38) adapté à cette section rectangulaire (68).

43. Endoprothèse selon la revendication 42, caractérisée en ce que l'espace intérieur (38) est limité par deux parois latérales (39, 40) opposées, pratiquement parallélépipédiques et parallèles par rapport au plan de pivotement (51).

44. Endoprothèse selon la revendication 43, caractérisée en ce que l'arbre (32) est fixé contre les parois latérales (39, 40) à une distance déterminée (63) de l'arête arrière (55), qui correspond pratiquement à la largeur de l'humérus (1) au point de jonction avec le boîtier (37).

45. Endoprothèse selon la revendication 43 ou 44, caractérisée en ce que les faces intérieures (69, 70) des parois latérales (39, 40), orientées vers l'espace intérieur (38), sont recouvertes de revêtements antifriction (42, 43) sur lesquels est monté, de manière à pouvoir glisser, l'élément de maintien (18) avec ses limites latérales (71, 72).

46. Endoprothèse selon la revendication 45, caractérisée en ce que les revêtements antifriction (42, 43) sont en polyéthylène.

47. Endoprothèse selon l'une quelconque des revendications 42 à 46, caractérisée en ce que l'espace intérieur (38) est ouvert vers le bas dans le sens du cubitus (2) et forme, dans le sens de la tige de l'humérus (36), une couverture du boîtier (41) sur laquelle est formée une butée (44) pour une arête supérieure (73) de l'élément de maintien (18) orientée vers ladite couverture du boitier.

48. Endoprothèse selon l'une quelconque des revendications 43 à 46, caractérisée en ce que les parois latérales (39, 40) sont munies d'une zone limite courbe (76) au moins sur les extrémités inférieures (74, 75) orientées vers le cubitus (2).

49. Endoprothèse selon la revendication 48, caractérisée en ce que la zone limite courbe (76) forme un arc de cercle dont le centre est situé sur la ligne médiane (64) de l'arbre (32).

50. Endoprothèse selon l'une quelconque des revendications 43 à 49, caractérisée en ce que la zone limite des parois latérales s'étend dans le sens du cubitus (2) au-dessus de la traverse transversale (8).

51. Endoprothèse selon l'une quelconque des revendications 43 à 50, caractérisée en ce que sur chacune des parois latérales (39, 40) est fixée une aile support (47, 48) qui s'écarte du boîtier (37), lesquelles s'étendent le long de la zone limite inférieure (76).

52. Endoprothèse selon la revendication 51, caractérisée en ce que la largeur des ailes support (47, 48) correspond à celle de l'espace intérieur (38).

53. Endoprothèse selon la revendication 51 ou 52, caractérisée en ce que chaque aile support (47, 48) présente une surface portante (77, 78) inférieure orientée vers le cubitus (2), laquelle présente transversalement par rapport à l'aile support (47, 48) une faible courbure (79) orientée dans le sens du cubitus (2).

54. Endoprothèse selon l'une quelconque des revendications 51 à 53, caractérisée en ce que les ailes support (47, 48) s'étendent sur un arc de 180°, qui part de l'arête arrière (55) et se poursuit le long de la zone limite (76).

55. Endoprothèse selon l'une quelconque des revendications 37 à 54, caractérisée en ce que l'arbre (32) est recouvert d'un revêtement antifriction (80).

56. Endoprothèse selon la revendication 55, caractérisée en ce que le revêtement antifriction (80) est en polyéthylène.

57. Endoprothèse selon l'une quelconque des revendications 37 à 56, caractérisée en ce que l'élément de maintien (18) est guidé sur l'arbre (32) par l'intermédiaire d'une vis de retenue (26), qui s'étend, d'une branche (21) vers l'autre branche (22), transversalement par rapport à la direction de l'arbre (32), et l'arbre (32) s'applique contre la surface dudit élément de maintien.

58. Endoprothèse selon la revendication 57, caractérisée en ce que la vis de retenue (26) traverse une rainure (50), qui est aménagée dans une surface de l'arbre (32) orientée vers l'élément de maintien (18), et la vis de retenue (26) englobe au moins une partie de sa surface.

59. Endoprothèse selon la revendication 58, caractérisée en ce que la rainure (50) limite une section angulaire de l'arbre (32) et la vis de retenue (26) s'étend le long d'une arête latérale de la section angulaire.

60. Endoprothèse selon l'une quelconque des revendications 43 à 59, caractérisée en ce que l'élément de maintien (18) s'engage à travers un alésage qui s'étend à travers l'arbre (32) et est fixé dans cet alésage, et en ce que l'arbre (32) est monté dans les parois latérales (39, 40) de manière à pouvoir pivoter.

61. Endoprothèse selon l'une quelconque des revendications 2 à 60, caractérisée en ce que la ligne médiane du radius (17) s'étend le long de la ligne médiane de la calotte (12) et, en position non tordue de l'avant-bras, s'étend parallèlement à la tige ulnaire.

62. Endoprothèse selon l'une quelconque des revendications 1 à 61, caractérisée en ce que la tête du radius (7) est montée dans la calotte (6) de manière à pouvoir pivoter.

63. Endoprothèse selon l'une quelconque des revendications 1 à 62, caractérisée en ce que la calotte (6) forme une courbure plane par rapport à la tête du radius (7).
